# EUROPEAN PATENT SPECIFICATION

(11) **EP 1 324 312 B1**
(45) Date of publication and mention of the grant of the patent: **13.02.2008**
(21) Application number: 02024930.6
(22) Date of filing: 06.11.2002
(51) Int. Cl.: G10H 1/34

(54) **Keyboard musical instrument having keys regulated with stable key balance pieces**
Tasteninstrument mit Tasten reguliert durch stabile ausbalancierende Teile
Instrument de musique à clavier avec des touches ajustées au moyen d'éléments d'équilibrage stables

(30) Priority: 08.11.2001 JP 2001342943; 29.11.2001 JP 2001363799; 15.03.2002 JP 2002072627
(43) Date of publication of application: 02.07.2003
(73) Proprietor: YAMAHA CORPORATION, Naka-ku Hamamatsu-shi Shizuoka-ken (JP)
(72) Inventor: Inoue, Satoshi, Hamamatsu-shi, Shizuoka-ken (JP)
(74) Representative: Emde, Eric

(56) References cited:
- DE-A- 19 538 267
- US-A- 4 679 477
- US-A- 4 899 631
- US-A- 5 585 582

## Description

### FIELD OF THE INVENTION

This invention relates to a keyboard musical instrument and, more particularly, to a keyboard musical instrument having keys regulated with key balance pieces.

### DESCRIPTION OF THE RELATED ART

The keyboard musical instruments are categorized in three groups. The first group is an electric or electronic keyboard musical instrument, and the second group is an acoustic keyboard musical instrument. Acoustic pianos, i.e., grand pianos and upright pianos are typical examples of the acoustic keyboard musical instrument. The third group is a compromise between the electric/ electronic keyboard musical instrument and the acoustic keyboard musical instrument. A silent piano is an example of the composite keyboard musical instrument between an acoustic piano and an electronic keyboard. The user has an option between acoustic piano tones and electronic tones. This means that the user can perform a passage through the acoustic piano tones or electronic tones.

In any sort of keyboard musical instrument, keys are indispensable component parts of the keyboard musical instrument, and serves as an interface between the keyboard musical instrument and users. The users specify the pitches of the tones to be produced through the keys. The acoustic keyboard musical instruments such as pianos give unique key-touch to the players, and the key-touch on the electronic keyboards is different from that of the acoustic keyboard musical instrument. Since the players are familiar with the key-touch on their keyboard musical instruments, the players, who usually play on the acoustic pianos, feel the keys of the electronic keyboards unfamiliar, and the players, who finger pieces of music on the electronic keyboard, feel the keys of the acoustic pianos strange. Professional pianists discriminate the key-touch of their own pianos from the key-touch of other pianos.

One of the factors of the key-touch is the appropriate difference between moment of force and the counter moment of force exerted on the respective keys. Another factor of the key-touch is the largeness of inertial of the respective keys. The keys are put on the balance rail so that the balance rail gives the fulcrums to the keys. Each key supports the action unit at the rear portion thereof, and exerts moment on the key. On the other hand, key balance pieces are, by way of example, embedded in the front portions of the keys in grand pianos, and exert the counter moment on the key. The key balance pieces are usually embedded in the rear portions of the keys in upright pianos. The moment is larger than the counter moment at the rest position so that the front portion floats over the key bed. When a pianist depresses the front portion, the front portion is sunk. The heavier the key balance pieces, the lighter the static key-touch. On the contrary, the heavier the key balance pieces, the heavier the dynamic key-touch Thus, both of the difference between the moment and the counter moment and the largeness of moment of inertial have the influence on the key-touch.

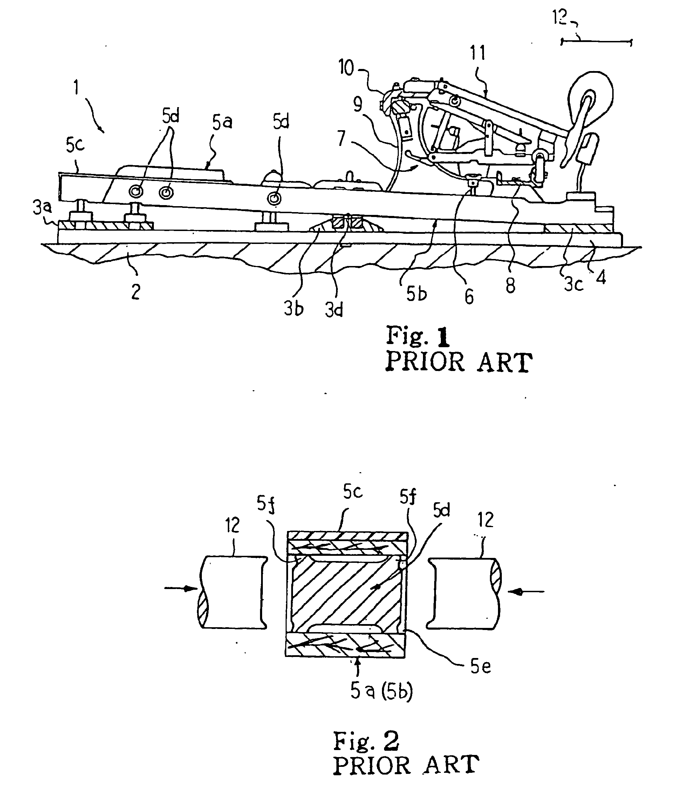

Figure 1 shows a typical example of the essential parts of the acoustic piano. In the following description term "front" is indicative of a position closer to a pianist, who sits on a stool for fingering, than a "rear" position. Term "fore-and-aft" direction is indicative of a virtual line between a front position and a corresponding rear position, and term "lateral" modifies the direction perpendicular to the fore-and-aft direction.

A keyboard 1 is mounted on a key bed 2, and a front rail 3a, a balance rail 3b and a back rail laterally extend on a key frame 4. Black and white keys 5a and 5b are put in parallel on the balance rail 3b, and extend in the fore-and-aft direction. The balance rail 3b gives the fulcrums 3d to the black and white keys 5a/ 5b so that the black and white keys 5a/ 5b are rotatable about the balance rail 3b. The front portions of the white keys 5b are covered with thin decorative plates 5c made of synthetic resin.

Capstan buttons 6 project from the rear portions of the black/ white keys 5a/ 5b, and are held in contact with action units 7. The action units 7 are rotatably connected to a whippen rail 8, which laterally extends over the rear portions of the arrays of black and white keys 5a/ 5b. The whippen rail 8 is supported by action brackets 9 on the key frame 4. A shank flange rail 10 is further supported by the action brackets 9, and laterally extends over the array of black and white keys 5a/ 5b. Hammers 1 are rotatably connected to the shank flange rail 10. The action units 7 are functionally connected to the hammers 11, and receive the weight of the associated hammers 11. When a pianist depresses a black/ white key 5a/ 5b, the associated action unit 7 is actuated so as to drive the hammer 11 for rotation. The jack of the action unit 7 escapes from the hammer 11, and the hammer starts the free ration. The hammer 1 strikes an associated string 12 at the end of the free rotation, and returns onto the action unit 7.

The weight of the hammer 1 and action unit 7 is applied through the capstan screw 6 to the rear portion of the associated black/ white key 5a/ 5b, and, accordingly, the moment is exerted on the black/ white key 5b in the clockwise direction. In order to partially cancel the moment, counter moment is exerted on the black/ white key 5a/ 5b, and key balance pieces 5d are embedded in the front portion of the black/ white key 5a/ 5b for the counter moment. The counter moment is smaller than the moment so that the front portion floats over the front rail 3a.

The key balance pieces 5d have a generally cylindrical shape, and both ends are exposed to the side surfaces of the black/ white key 5a/ 5b. The key balance pieces 5d are of the order of 10 millimeters in diameter. The key balance pieces 5d are also embedded in the other black/ white keys 5a/ 5b. The key balance pieces 5d are made of lead. The key balance pieces 5d are embedded in the black/ white keys 5a/ 5b as follows. First, through-holes 5e are formed in the front portions of the black and white keys 5a/ 5b (see figure 2). Cylindrical lead pieces are prepared, and have a diameter less than the diameter of the through-holes 5e. The cylindrical lead pieces are smoothly inserted into the through-holes 5e. A pair of bits 12 is pressed against the exposed surfaces of each cylindrical lead piece. The cylindrical lead piece is plastically deformed, and both end portions are radially spread. As a result, the deformed end portions 5f are tightly fit to the inner surfaces of the black/white key 5a/ 5b. Thus, the key lead pieces 5d are anchored to the associated black/ white key 5a/ 5b by means of the deformed end portions 5f.

The first reason why the lead is used is that the lead has the large specific gravity. The specific gravity of the lead is 11.34, and is one of the heaviest industrial metals. This means that small lead pieces give rise to large moment, and small space such as the narrow through-holes 5e are merely required for the small lead pieces. The key balance pieces 5d of lead can exert large counter moment on the black and white keys 5a/ 5b. In other words, a tuner can adjust the black/ white key 5a/ 5b to the most desirable key-touch between the light key-touch and the heavy key-touch.

Another reason why the lead is used is that the lead is rich in plasticity. As described hereinbefore, the key balance pieces 5d are anchored to the associated black/ white key 5a/ 5b through the plastically deforming process. If the cylindrical balance pieces are made of hard metal, large force is to be exerted on the both end portions, and the deformed end portions are strongly pressed against the inner surfaces. The black/ white keys 5a/ 5b are made of wood so that the wooden key 5a/ 5b are liable to be broken. Moreover, the hard metal pieces are less fit to the inner surfaces of the black/ white key 5a/5b, and tend to be dropped out.

Yet another reason is that the lead is economical. Although gold and platinum are large in specific gravity and rich in plasticity, they are so expensive that the people can not purchase the acoustic piano. The lead is not expensive, and the manufacturer reduces the production cost of the acoustic piano.

Thus, the key balance pieces 5d of lead are preferable for the wooden keys 5a/ 5b. However, the lead is detrimental to health, and contaminates the environment. Several alternate materials have been proposed.

One of the alternate materials is disclosed in Japan Patent Application laid-open 2001-142454. The key balance pieces disclosed in the Japan Patent Application laid-open are made of a sort of composite material. The composite material contains resilient material and non-lead metal. The resilient material is mixed with the non-lead metal, and the composite material is shaped in the cylindrical configuration. The resilient material enhances the elasticity of the composite material.

The key balance pieces of the composite material are embedded in the key as follows. First, the through-holes are formed in the wooded key, and cylindrical pieces are tightly inserted into the through-holes. The cylindrical pieces are pressed against the inner surfaces of the through-holes by virtue of the elasticity, the key balance pieces are anchored to the wooden key. Namely, the key balance pieces of the composite material are embedded in the wooden key as similar to the above-described prior art keys 5a/ 5b.

Thus, the black/white keys 5a/ 5b only rely on the elasticity of the composite material. However, the through-holes are not always the adjusted to the target diameters, and the wooded keys tend to be shrunk for a long service time. In case where the through-holes have narrow through-holes, the wooden keys are liable to be cracked. On the other hand, if the through-holes are too wide, the elastic force is insufficient to keep the cylindrical pieces in the through-holes. When a pianist depresses the key, the key balance pieces chatter in the holes. If the looseness is serious, the key balance piece is dripped out. Thus, a problem is encountered in the key balance pieces disclosed in Japan Patent Application laid-open No. 2001-142454 in that the key balance pieces are not tightly fit into the keys.

Another key balance piece is disclosed in Japan Patent Application laid-open No. 2001-147685. The key balance piece disclosed in the Japan Patent Application laid-open is a combination between a tubular member and a rigid column. The tubular member is made of resilient material, and the rigid column is made of composite material. Plural sorts of non-lead material, which are different in specific gravity, are mixed in such a manner that the composite material is adjusted to a target value of the specific gravity. The rigid columns are received in the resilient tubular members, and the resilient tubular members are inserted into the holes formed in the key through a press fitting.

The resilient tubular members are well fit in the holes. However, the rigid columns are merely held in the resilient tubular members by the agency of the resiliency of the resilient tubular members. In case where the rigid columns are finished smaller than the design drawing, the rigid columns are liable to be dropped out from the resilient tubular members.

Yet another key balance pieces are disclosed in Japan Patent Application laid-open No. 2001-154661. The key balance pieces disclosed in the Japan Patent Application laid-open is made of composite material, and small semi-spherical projections are formed on the outer surface of the key balance pieces. The composite material consists of plural sorts of non-lead metal and synthetic resin. The key balance pieces are inserted into the holes formed in the key through a press fitting, and the small projections are caught on the inner surfaces. Although most of the small projections are held in contact with the inner surfaces of the key, the semi-spherical projections have round contact surfaces, and are liable to slide on the inner surfaces of the key. In other words, the semi-spherical projections are hardly caught on the inner surfaces of the key. For this reason, the key balance pieces are liable to be dropped out.

Still another key balance piece is disclosed in Japan Patent Application laid-open No. 2001-175248. The key balance piece disclosed in the Japan Patent Application laid-open is made of composite material. Metal or alloy such as copper, brass iron and tungsten are mixed with fluid material such as thermosetting synthetic resin, thermoplastic synthetic resin, fusible alloy and adhesive compound in organic compound series. The metal or alloy is mixed with the fluid material, and the mixture is poured into cavities formed in a key. The mixture is solidified so that the key balance pieces are embedded in the key. However, it is not easy to fill the cavities with the mixture. If the mixture is too much, the key is contaminated with the residue. On the other hand, if the mixture is short, the key balance pieces are liable to be dropped out after the solidification.

Yet another key balance piece is disclosed in Japan Patent Application laid-open No. 2001-195056. The key balance piece disclosed in the Japan Patent Application laid-open consists of a tubular member and a rigid column. The tubular member is made of heat contracting synthetic resin, and the rigid column is made of non-lead metal. The rigid column is received in the tubular member, and the tubular member is inserted into the holes formed in the key through the press fitting. Although the tubular member are made of the heat contracting synthetic resin, the key balance piece consists of the combination of the tubular member and the rigid column as similar to the key balance piece disclosed in Japan Patent Application laid-open No. 2001-142454. For this reason, the key balance pieces are also unstable, and are liable to be dropped out.

The key balance pieces disclosed in those documents are embedded in the keys through the press fitting or solidification of fluid material. However, the key balance pieces are held in the holes or cavities by the agency of friction. For this reason, the key balance pieces are liable to be dropped out from the keys.

US-A-4,679,477 was used as a basis for the preamble of claims 1 and 9 and discloses a percussive action silent electronic keyboard which provides electrical input signals for electronic music synthesis equipment. The keyboard includes a housing, and a keyboard array of a plurality of depressable pivoted playing keys adjacently arranged as a musical keyboard. Each key communicates with a pivoted silent hammer in a cam and follower arrangement. A stop is provided for stopping the momentum of each silent hammer which is caused to move about its pivot by following a camming surface of its corresponding key resulting from depressing of the key during playing action. An electrical switch provided for each key, and the switch is responsive to the playing action of the key for generating and supplying electrical signals indicative of the action to the electronic music synthesis equipment with which said keyboard may be used to generate music.

Key balancers are also used for the keys incorporated in the electronic keyboards, and are similar to those for the keys of the acoustic pianos. This means that the key balancers are liable to be dropped out from the keys.

### SUMMARY OF THE INVENTION

It is therefore an important object of the present invention to provide a keyboard musical instrument, which has keys regulated with stable balancers.

It is another object of the present invention to provide a process for fabricating a key which is simple and conducive to reduction of the production cost.

In accordance with one aspect of the present invention, there is provided a keyboard musical instrument for generating tones as set forth in claim 1. Preferred embodiments of the present invention may be gathered from the dependent claims.

In accordance with another aspect of the present invention, there is provided a process for fabricating a key incorporated in a keyboard musical instrument comprising the steps of a) preparing a bar and a weight piece, and b) securing the weight piece to the bar, wherein the step a) includes the sub-step of forming a substantially straight through-hole in the bar and the weight piece into a shape having at least a substantially straight tube portion roughly equal in diameter to the substantially straight through-hole and a core portion disposed in the substantially straight tube portion, and the step b) includes the sub-steps of b-1) inserting the weight piece into the substantially straight through-hole and b-2) caulking both end portions of the substantially straight tube portion to the side portions of said bar so that both of the both end portions and the side portions are flared.

### BRIEF DESCRIPTION OF THE DRAWINGS

The features and advantages of the keyboard musical instrument will be more clearly understood from the following description taken in conjunction with the accompanying drawings, in which
Fig. 1 is a side view showing the essential parts of the prior art acoustic piano,
Fig. 2 is a cross sectional view showing the key lead piece embedded in the key,
Fig. 3 is a perspective view showing a white key incorporated in an acoustic piano according to the present invention,
Fig. 4 is a cross sectional view taken along line A-A of figure 3 and showing a key balancer embedded in the white key,
Fig. 5 is a perspective view showing the first modification of the key balancer according to the present invention,
Fig. 6 is a front view showing the second modification of the key balancer according to the present invention,
Fig. 7 is a perspective view showing the third modification of the key balancer according to the present invention,
Figs. 8A and 8B are a cross sectional view and a perspective view showing the fourth modification of the key balancer according to the present invention,
Fig. 9 is a perspective view showing a white key incorporated in another acoustic piano according to the present invention,
Fig. 10 is a cross sectional view taken along line B-B of figure 9 and showing the structure of a key balancer embedded in the white key,

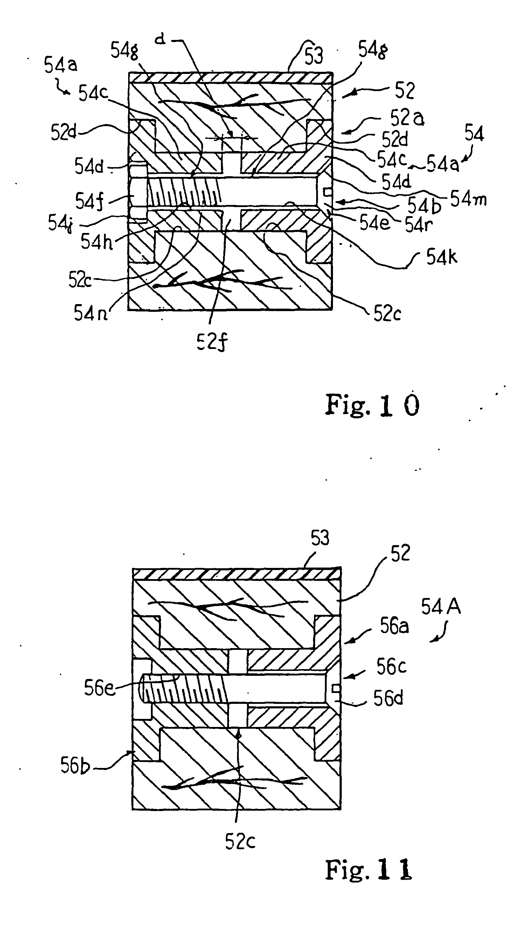

Fig. 1 1 is a cross sectional view showing the first modification of the key balancer shown in figure 10,

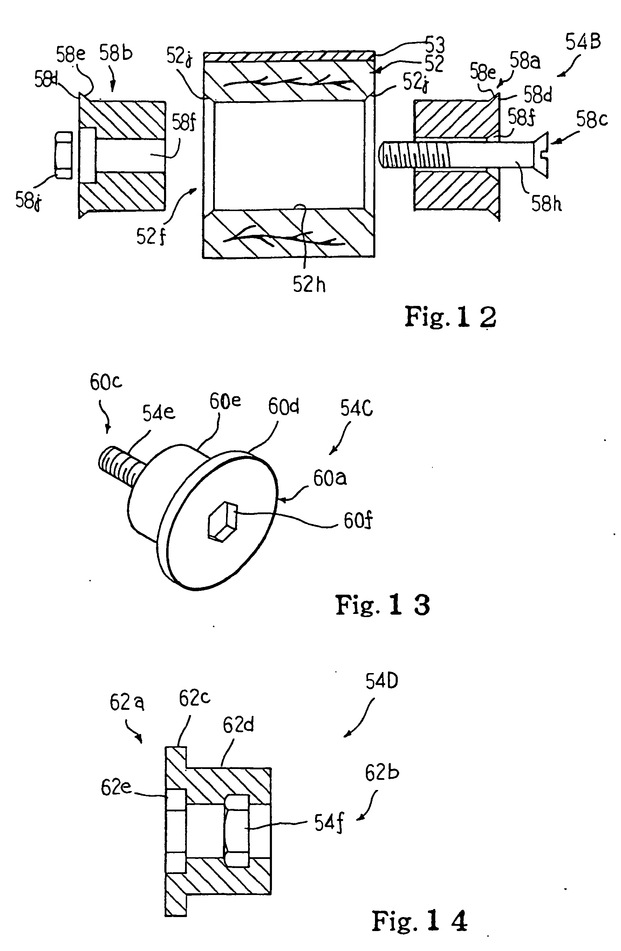

Fig. 12 is a cross sectional view showing the second modification of the key balancer shown in figure 10,
Fig. 13 is a cross sectional view showing the third modification of the kev balancer shown in figure 10,
Fig. 14 is a cross sectional view showing the fourth modification of the key balancer shown in figure 10,

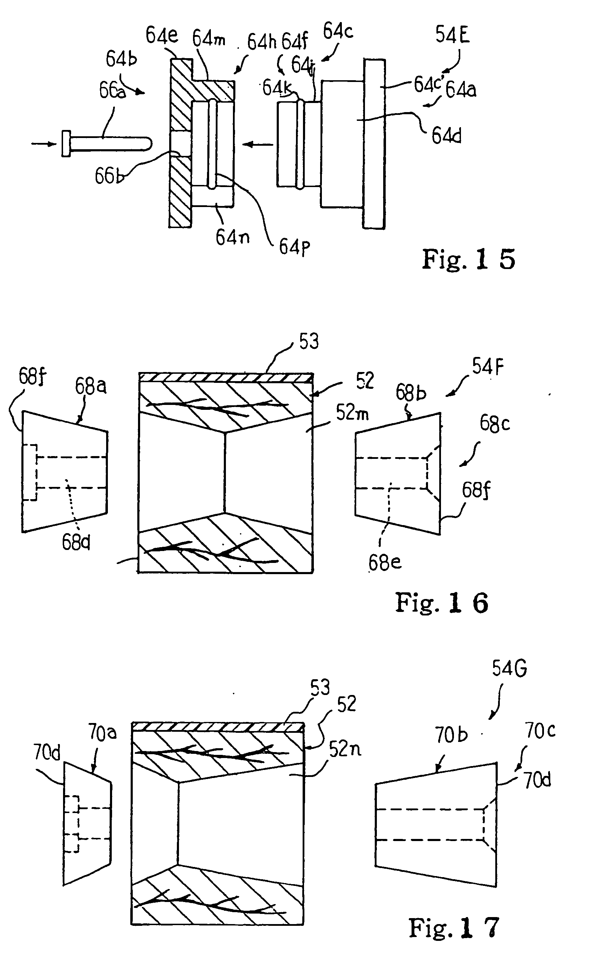

Fig. 15 is a cross sectional view showing the sixth modification of the key balancer shown in figure 10,
Fig. 16 is a cross sectional view showing the seventh modification of the key balancer shown in figure 10,
Fig. 17 is a cross sectional view showing the eighth modification of the key balancer shown in figure 10,

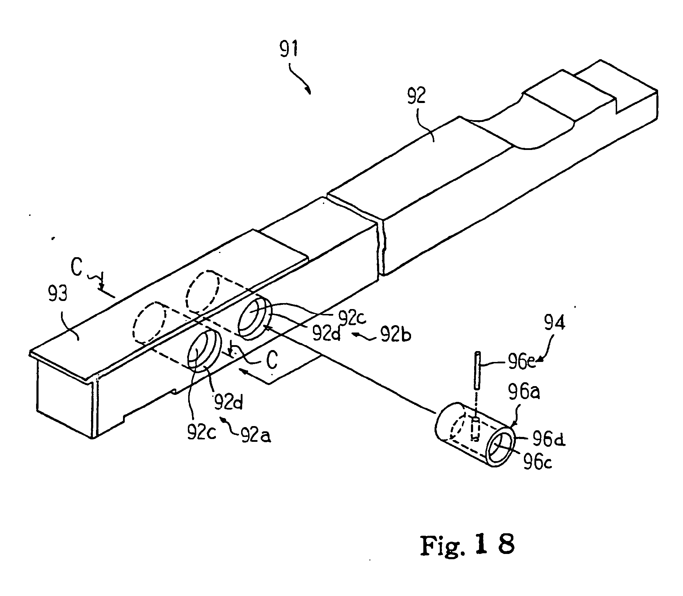

Fig. 18 is a perspective view showing a white key incorporated in yet another acoustic piano according to the present invention,

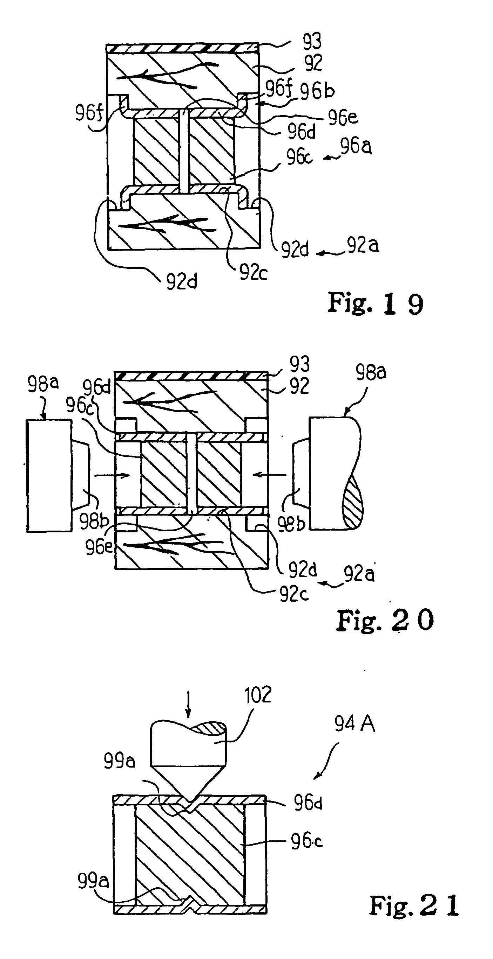

Fig. 19 is a cross sectional view taken along line C-C of figure 18 and showing a key balancer in the white key,
Fig. 20 is a cross sectional view showing caulking bits used for forming a fastener,
Fig. 21 is a cross sectional view showing the first modification of the kev balancer, and

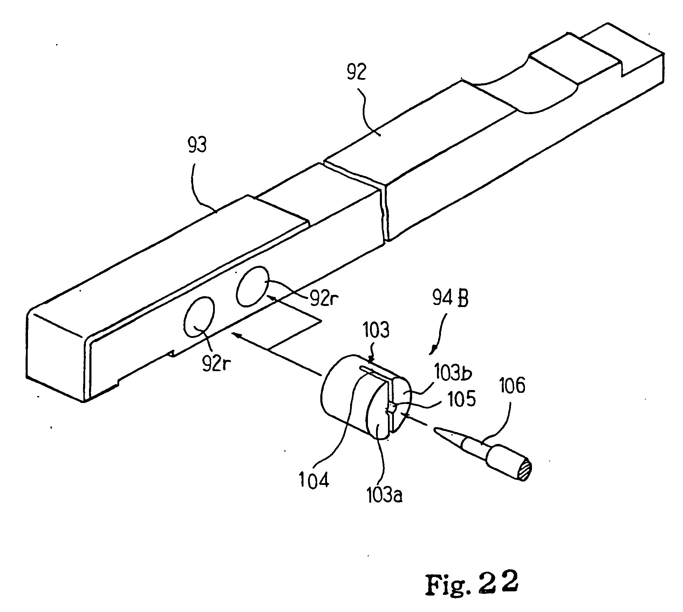

Fig. 22 is a perspective view showing the second modification of the key balancer.

### DESCRIPTION OF THE PREFERRED EMBODIMENTS

### First Embodiment

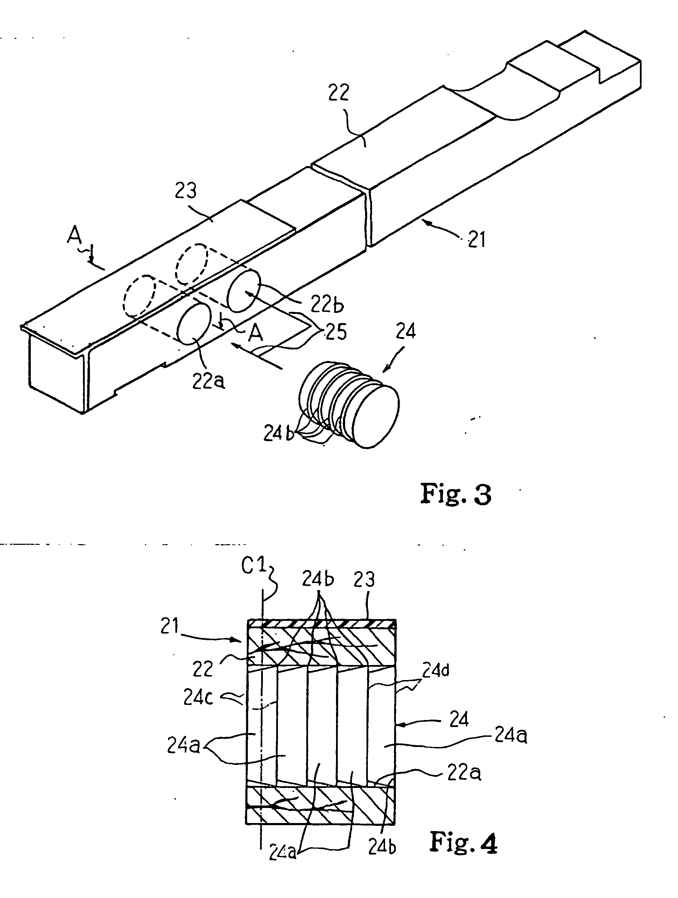

Figure 3 illustrates a key 21 forming a part of a keyboard, which in turn is incorporated in an acoustic piano. The acoustic piano is similar to that shown in figure 1 except the keys 21. For this reason, description is focused on the key 21, and the other component parts are specified by using references designating corresponding parts in figure 1.

The key 21 serves as a white key, and comprises a wood bar 22, a decorative plate 23 and key balancers 24. The wood bar 22 is made of Japanese spruce, which belongs to silver fir. Although a front portion and a rear portion are shown in figure 3, a vertical hole (not shown) is formed in a middle portion of the wood bar 22, and a balance pin (not shown) passes through the vertical hole. The balance pin gives the fulcrum 3d to the white key 2 1 placed on the balance rail 3b.

The upper surface and front end surface of the front portion of the white key 21 are covered with the decorative plate 23. The decorative plate 23 is like an angle, and is adhered to the wood bar 22. The decorative plate 23 is made of synthetic resin, and the synthetic resin is colored in white.

Two through-holes 22a and 22b are formed in the front portion of the wood bar 22. The through-holes 22a/ 22b are in parallel, and each of the through-holes 22a/ 22b is open at both ends thereof on the side surfaces of the wood bar 22 to the outside. The key balancers 24 are inserted into the through-holes 22a/ 22b so as to exert the counter moment on the white key 21.

The key balancers 24 have a contour like an aggregate of frusto-conical pieces 24a as shown in figure 4. The pieces of frustum of cone, i.e., frusto-conical pieces 24 have respective centerlines aligned with one another. The frusto-conical piece 24a is asymmetrical with respect to a cross section passing through the middle point on the centerline and parallel to the top and bottom circular planes thereof. For example, the leftmost frusto-conical piece 24a is asymmetrical with respect to the cross section C1 passing through the middle point on the centerline. The bottom circular plane is wider than the top circular plane so that sharp ridges 24b take place along the centerline of the key balancer 24 at regular intervals. Although the top circular planes 24c are narrower than the cross sections of the through-holes 22a/ 22b, the bottom circular planes 24d are slightly wider than the cross section so that the key balancers 24 are inserted into the through-holes 22a/ 22b through a press fitting as indicated by arrows 25. The sharp ridges 24b are preferable for the key balancers 24. However, the diameter of the sharp ridges are to be slightly larger than the inner diameter of the through-holes 22a/ 22b. If the sharp ridges 24b have a diameter much larger than the inner diameter of the through-holes 22a/ 22b, the wood bar 22 would be cracked during the press fitting. Although the sharp ridges 24b make the through-holes 22a/ 22b wider than the cross section of the original through-holes 22a/ 22b during the press fitting, the key balancers 24 are not pulled out from the through-holes 22a/22b, because the sharp ridges 24b bite the inner wall portions of the wood bar 22. Moreover, the key balancers 24 do not proceed further into the through-holes 22a/ 22b, because the remaining parts of the through-holes 22a/ 22b still have the cross section smaller in diameter than the sharp ridges 24b.

The key balancer 24 is made of heavy metal except harmful metal such as lead and mercury. The heavy metal available for the key balancers 24 are, by way of example, iron, brass, tungsten and sintered metal. Composite material is also available for the key balancers 24. The composite material contains the heavily metal and synthetic resin. Although any sort of non-harmful metal is available for the key balancers 24, tungsten is preferable. Tungsten has the specific gravity of 19.24, and is heavier than lead. Even though the synthetic resin is mixed with tungsten, the composite material has the specific gravity as large as lead. The composite material is to be larger in hardness than the wood bar 22. The synthetic resin available for the key balancers 24 is, by way of example, thermosetting resin in the urethane series, polyester series, epoxy series, phenol series, urea series and melamine series or thermoplastic resin in the ABS (Acrylonitrile- Butadiene- Styrene) series and acrylic resin series. It is preferable to increase the amount of heavy metal of the composite material, because the key balancers 24 exert large counter moment on the key 21. In case where the key balancers 24 are made of iron, the exposed surfaces of the key balancers 24 are to be preserved.

The key 21 is assembled as follows. The through-holes 22a/ 22b are formed in the wood bar 22, and the centerline of each key balancer 24 is roughly aligned with the centerline of associated one of the through-holes 22a/ 22b. The key balancer 24 is partially inserted into the associated through-hole 22a/ 22b. However, the first sharp ridge 24b does not allow the key balancer 24 to proceed into the through-hole 22a/ 22b. Then, the worker presses the key balancer 24 to the wood bar 22. The key balancer 24 is pushed into the through-hole 22a/ 22b, and the sharp ridges 24b are strongly caught on the inner surface of the wood bar 22. The worker repeats the press fitting for the other key balancer 24. It is difficult to pull out the key balancers 24 from the through-holes 22a/ 22b, because the sharp ridges 24b bite the inner wall portions defining the wood bar 22.

In this instance, the sharp ridges 24b serve as anchors. As will be understood from the foregoing description, the anchors, i.e., the sharp ridges 24b strongly bite the inner wall portions of the wood bar 22, and prevent the key balancers 24 from dropping out from the key 21.

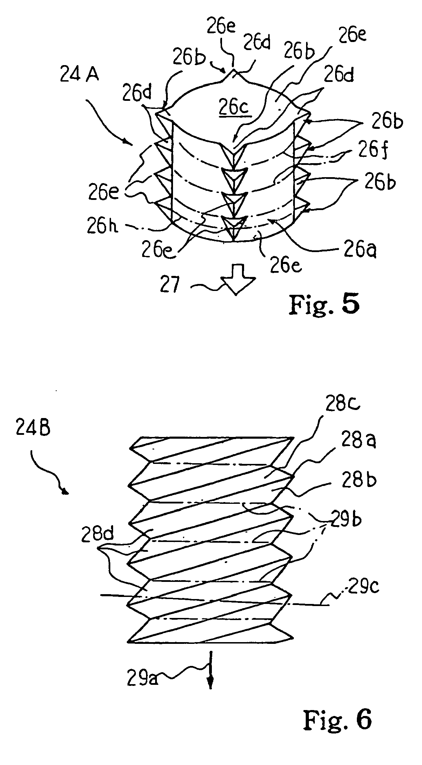

Figure 5 shows the first modification 24A of the key balancer 24. The key balancer 24A is made of the heavy metal except the harmful metal such as lead and mercury. Iron, brass, tungsten and sintered metal are available for the key balancer 24A. The composite material, which contains the non-harmful metal and synthetic resin, is also available for the key balancer 24A.

The key balancer 24A has a trunk portion 26a and sharp teeth 26b. The sharp teeth 26b are arranged in four columns, and each column of sharp teeth 26b is 90 degrees spaced from the adjacent columns of sharp teeth 26b. The sharp tooth 26b has a pyramid shape. The bottom surfaces 26d of the pyramid shaped teeth 26b are coplanar with or in parallel to the end surfaces 26c of the trunk portion 26a so that sharp tips 26e take place.

The key balancer 24A is imaginarily dividable into plural parts 26e as indicated by phantom lines 26f. Each part 26e includes four sharp teeth 26b 90 degrees spaced from one another, and is asymmetrical with respect to a virtual cross section 26h.

The key balancer 24A is inserted into the through-holes 22a/ 22b as indicated by arrow 27. The sharp tips 26e bite the inner wall portions of the wood bar 22 so that the key balancers 24A are stable in the through-holes 22a/ 22b. In the first modification, the sharp tips 26e serve as anchors.

Figure 6 shows the second modification 24B of the key balancer 24. The key balancer 24B is made of the heavy metal except the harmful metal such as lead and mercury. Iron, brass, tungsten and sintered metal are available for the key balancer 24B. The composite material, which contains the non-harmful metal and synthetic resin, is also available for the key balancer 24B.

The key balancer 24B has a generally column shape, and a spiral ridge 28a is formed in the outer surface portion. The spiral ridge 28a is defined by two spiral surfaces 28b and 28c. In this instance, the spiral ridge 28a has a triangular cross section. However, the key spiral ridge 28a may have a rectangular cross section or another polygonal cross section. The spiral ridge 28a is either left-handed or right-handed. If the key balancer 24B is inserted into the through-holes formed in the key in the direction indicated by arrow 29a, it is preferable that the spiral surface 28b inclines larger in angle than the other spiral surface 28c, because the spiral ridge 28a strongly bites the inner surface portion of the key in the motion reverse to the arrow 29a.

The key balancer 24B is also imaginarily dividable into plural parts 28d as indicated by phantom lines 29b, and each part 28d is asymmetrical with respect to a virtual cross section 29c at the mid point of the centerline and parallel to the both end surfaces of the key balancer 24B.

A worker embeds the key balancers 24B in the key as follows. First, the worker forms through-holes 22a/ 22b in the wood bar 22, and roughly aligns the centerline of the key balancer 24B with the centerline of the through-hole 22a. The worker drives the key balancer 24B for rotation. Then, the key balancer 24B advances into the through-hole 22a through the screw-motion. The worker repeats the operations for the other key balancers 24B. Even if the key balancer 24B is rearward pulled, the sharp ridge 28a bites the inner surface portion of the key 21, and prevents the key balancer 24B from being dropped out.

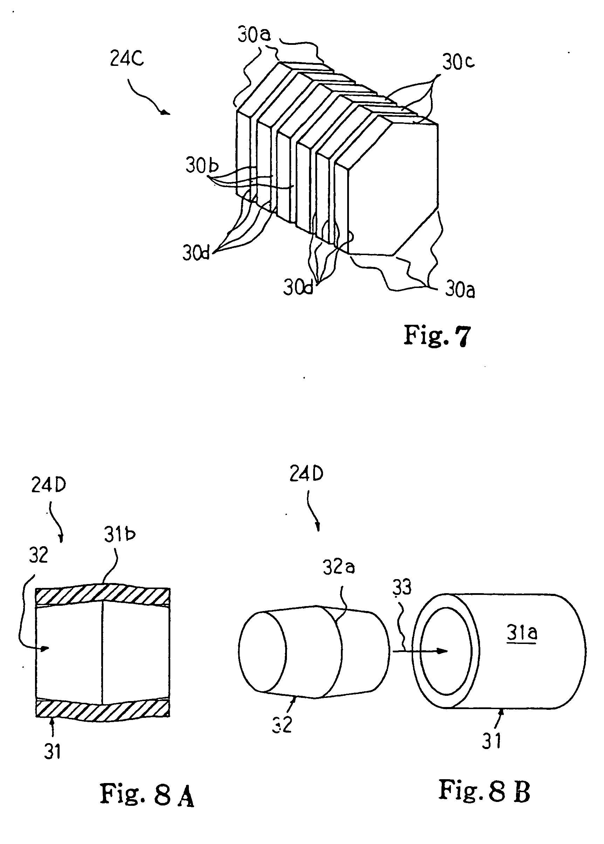

Figure 7 shows the third modification 24C of the key balancer 24. The key balancer 24C is also made of the heavy metal except the harmful metal such as lead and mercury. Iron, brass, tungsten and sintered metal are available for the key balancer 24C. The composite material, which contains the non-harmful metal and synthetic resin, is also available for the key balancer 24C.

The key balancer 24C has a generally a hexagonal column, and, accordingly, six ridges 30a take place along the centerline of the hexagonal column. Plural grooves 30b are formed in the hexagonal column so that plural hexagonal parts 30c are spaced from one another along the centerline at intervals. The grooves 30b do not reach the centerline so that the plural hexagonal parts 30c are integral. The plural hexagonal parts 30c have sharp peripheries 30d.

The key balancers 24C are embedded in the key 21 as follows. A worker forms the through-holes 22a/ 22b in the wood bar 22, and roughly aligns the centerline of the key balancer 24C with the centerline of the through-hole 22a/ 22b. The worker forcibly inserts the key balancer 24C into the through-hole 22a/ 22b through a press fitting. Even if the key balancer 24C is rearward pulled, the sharp peripheries 30d bite the inner surface portion of the key 21, and the key balancer 24C is hardly moved in the rearward direction. The six ridges 30a prevent the key balancers 24C from rotation in the through-holes 22a/ 22b.

Figures 8A and 8B show the fourth modification 24D of the key balancer 24. The key balancer 24D is a combination of a resilient tubular member 31 and a weight piece 32. The weight piece 32 is made of the heavy metal except the harmful metal such as lead and mercury. Iron, brass, tungsten and sintered metal are available for the key balancer 24D. The composite material, which contains the non-harmful metal and synthetic resin, is also available for the key balancer 24D.

The weight piece 32 has a shape like a barrel. The weight piece 32 is increased in cross section from one end toward a middle section along the centerline thereof, and is decreased in cross section from the middle section to the other end. Thus, the weight piece 32 is tapered from the middle section toward both ends, and a ridge 32a is formed. The maximum diameter on the middle section is slightly larger than the diameter of the through-holes 22a/22b.

On the other hand, the resilient tubular member 31 is approximately equal in diameter to the through-holes 22a/ 22b. While the weight piece 32 is out of the tubular member 31, the tubular member 31 has a straight outer surface 31a as shown in figure 8B. However, when the weight piece 32 is received in the tubular member 31, the resilient tubular member 31 is partially bulged as shown in figure 8A.

The key balancer 24D is embedded in the key 21 as follows. First the through-holes 22a/ 22b are formed in the wood bar 22, and the resilient tubular members 31 are inserted into the through-holes 22a/ 22b, respectively. The worker roughly aligns the centerline of the weight piece 32 with the centerline of the resilient tubular member 31. The worker forcibly inserts the weight pieces 32 into the resilient tubular members 31 through a press fitting. The resilient tubular members 31 are partially bulged due to the ridge 32a, and are strongly pressed to the inner surfaces of the wood bar 22. The bulged portion 31b of the resilient tubular member 31 prevents the key balancer 24D from being dropped out. In this instance, the bulged portion 31b and ridge 32a serve as an anchor. The weight piece or pieces 32 may be used as the fifth modification of the key balancer 24.

As will be understood from the foregoing description, the key balancers 24/ 24A/ 24B/ 24C/ 24D have the anchors 24b, 26b, 28a, 30d and 31b/ 32a so that the key balancers 24/ 24A/ 24B/ 24C/ 24D are hardly dropped out from the through-holes without any adhesive compound. Especially, the anchors 24b, 26b and 30b are asymmetrical with respect to the virtual cross sections C1/ 26h/ 29c of the unit portions 24a, 26e and 28d, and, accordingly, are sharp. This results in that the sharp anchors 24b/ 26b/ 30d strongly bite the inner surface portions of the wood bars 22. Thus, the anchors 24b/ 26b/ 30d keep the key balancers 24/ 24A/ 24B stable in the through-holes.

The key balancers 24/ 24A/ 24B/ 24C/ 24D are inserted into the through-holes 22a/ 22b through the press fitting or screw motion. The assembling work is simple and easy. This results in reduction of the production cost.

The key balancers 24/ 24A/ 24B do not contain any harmful element, and are desirable from the viewpoint of the human health and safety environment.

### Second Embodiment

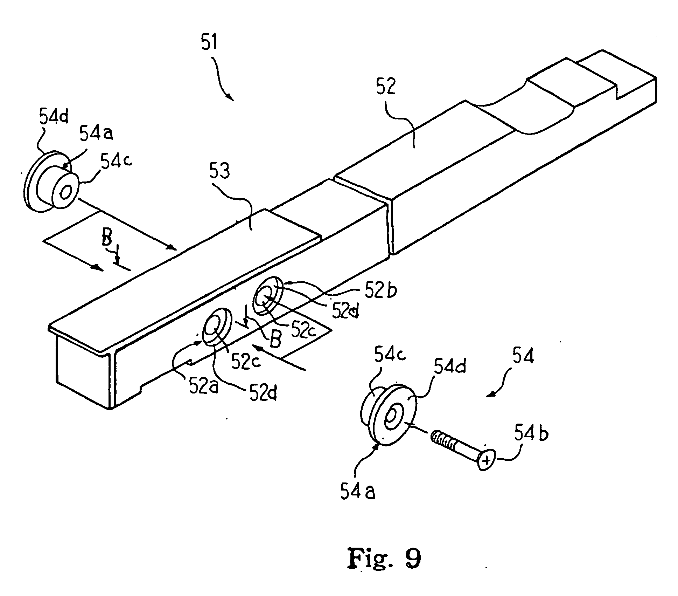

Figure 9 shows a key 51 forming a part of a keyboard, which is incorporated in an acoustic piano. The acoustic piano is similar to that shown in figure 1 except the keys 51. For this reason, description is focused on the key 51, and the other component parts are specified by using references designating corresponding parts in figure 1.

The key 51 serves as a white key, and comprises a wood bar 52, a decorative plate 53 and key balancers 54. The wood bar 52 is made of Japanese spruce, which belongs to silver fir. Although a front portion and a rear portion are shown in figure 9, a vertical hole (not shown) is formed in a middle portion of the wood bar 52, and a balance pin (not shown) passes through the vertical hole. The balance pin gives the fulcrum 3d to the white key 51 placed on the balance rail 3b.

The upper surface and front end surface of the front portion of the white key 51 are covered with the decorative plate 53. The decorative plate 53 is like an angle, and is adhered to the wood bar 52. The decorative plate 53 is made of synthetic resin, and the synthetic resin is colored in white.

Two through-holes 52a and 52b are formed in the front portion of the wood bar 52. The through-holes 52a/ 52b are in parallel, and each of the through-holes 52a/ 52b is open at both ends thereof on the side surfaces of the wood bar 52 to the outside. Each of the through-holes 52a/ 52b has a narrow portion 52c and wide portions 52d. As will be seen in figure 10, the wide portions 52d are exposed to the side surfaces of the wood bar 52, and are formed on both sides of the narrow portion 52c. In this instance, the wide portions 52 are equal in depth and diameter to one another. The key balancers 54 are embedded in the through-holes 52a/ 52b so as to exert the counter moment on the white key 51.

Each of the key balancer 54 includes weight pieces 54a and a fastener 54b. The fastener 54b serves as an anchor. Each of the weight pieces 54a has a stem portion 54c and a head portion 54d. The stem portion 54c is approximately equal in diameter to the narrow portion 52c, and the length of the stem portion 54c is shorter than a half of the length of the narrow portion 52c. The head portion 54d has the diameter and thickness approximately equal to the diameter and depth of the wide portion 52d. The thickness of the head portions 54d may be less than the depth of the wide portions52d. The weight pieces 54a are inserted into each through-hole 52a/ 52b from both side surfaces. The tolerance between the head portions 54d and the wide portions 52d is fairly large in value so that head portions 54d are rather loosely received in the wide portions 52d, respectively. Similarly, the tolerance between the stem portions 54c and the narrow portion 52c is large in value, and the stem portions 54c loosely extend in the narrow portion 52c. The head portions 54d are substantially coplanar with the side surfaces of the wood bar 52. The stem portions 54c are spaced from one another in the narrow portion 52c. Thus, a gap 52f takes place between the stem portions 54c as shown in figure 10.

A through-hole 54g is formed along the centerline of the weight piece 54a. The through-hole 54g formed in one of the weight piece 54a has a narrow portion 54h/ 54k and a hexagonal wide portion 54j, and the hexagonal wide portion 54j and narrow portion 54h form a step. On the other hand, the through-hole 54g formed in the other weight piece 54a has a narrow portion 54h and a frusto-conical portion 54m. The narrow portion 54h is equal in diameter to the narrow portion 54k.

The fastener 54b includes a flat head bolt 54e and a hexagon nut 54f. The flat head bolt 54e has a threaded stem 54n, which is narrower than the narrow portions 54h/ 54k, and a head 54r. The hexagonal nut 54f has the thickness equal to the depth of the hexagonal wide portion 54j so that the hexagonal nut 54f is received in the hexagonal wide portion 54j without projecting from the side surface of the wood bar 52. On the other hand, the head 54r is received in the frusto-conical portion 54m, and the threaded stem 54n extends in the narrow portions 54k/ 54h. The hexagon nut 54f is engaged with the threaded stem 54n, and the weight pieces 54a are fastened to the wood bar 52 by means of the flat head bolt 54e and hexagon nut 54f. Even if the flat head bolt 54e is deeply screwed into the hexagon nut 54f, the distance d is not decreased to zero, and the hexagon nut 54f is not loosened.

Even though the weight pieces 54a are loosely fit to the wood bar 52, the fastener 54b keeps the weight pieces 54a stable in the key 51. Moreover, one of the weight pieces 54a are identical with the other weight piece 54a, and both weight pieces 54a are made of certain material described hereinafter in detail. Although the head portion 54r is different in weight from the hexagon nut 54f, the weight pieces 54a have the weight much greater than the difference in weight between the head portion 54r and the hexagon nut 54f, and the key 51 is never twisted due to the unbalance. If the flat head bolt is replaced with a hexagonal headed bolt, the different in weight is minimized.

The weight pieces 54a are made of heavy metal except harmful metal such as lead and mercury. The heavy metal available for the weight pieces 54a is, by way of example, iron, brass, tungsten and sintered metal. Composite material is also available for the weight pieces 54a. The composite material contains the heavily metal and synthetic resin. Although any sort of non-harmful metal is available for the weight pieces 54a, tungsten is preferable. Tungsten has the specific gravity of 19.3, and is heavier than lead. Even though the synthetic resin is mixed with tungsten, the composite material has the specific gravity as large as or larger than lead. The composite material is to be larger in hardness than the wood bar 52. The synthetic resin available for the weight pieces 54a is, by way of example, thermosetting resin in the urethane series, polyester series, epoxy series, phenol series, urea series and melamine series or thermoplastic resin in the ABS (Acrylonitrile- Butadiene- Styrene) series and acrylic resin series. It is preferable to increase the amount of heavy metal of the composite material, because the weight pieces 54a exert large counter moment on the key 51. In case where the weight pieces 54a are made of iron, the exposed surfaces of the weight pieces 54a are to be preserved.

As will be understood from the foregoing description, the acoustic piano according to the present invention includes the key 51, in which the fasteners 54b keeps the key balancers 54 stable. The fastener 54b, i.e., anchors permit a worker to embed the weight key balancers 54 in the wood bar 52 easily. In case where the fastener 54b is implemented by the combination of the bolt 54e and nut 54f, the worker easily disassemble the key balancers 54 from the wood bar 52. The key balancers 54 are not made of any harmful material so that the key balancers are preferable from the viewpoint of the health and environment.

There are various modifications. Figure 11 shows the first modification 54A of the balancer 54. The key balancer 54A also includes weight pieces 56a/ 56b and a fastener 56c. The weight piece 56a is same as the weight piece 54a. However, the other weight piece 56b and fastener 56c are different from the weight piece 54a and fastener 54b, respectively. The fastener 56c is implemented by only the flat-head bolt 56d, and the female screw 56e is formed in the inner surface portion of the weight piece 56b. The flat head bolt 56d is engaged with the female screw 56e so that the weight pieces 56a/56b are fastened to the wood bar 52. The fastener 56c is constituted by only one part 56d so that the production cost is reduced.

Fig. 12 shows the second modification 54B of the key balancer 54. The key balancer 54B includes weight pieces 58a/ 58b and a fastener 58c. The fastener 58c serves as an anchor. A through-hole 52f is formed in the wood bar 52, and has a straight portion 52h and tapered portions 52j. The tapered portions 52j are formed on both ends of the straight portion 52h, and are exposed to the side surfaces of the wood bar 52. The weight pieces 58a/ 58b have respective brim portions 58d, which have tapered surfaces 58e, respectively. The weight pieces 58a/ 58b are received in the through-holes 52f, and the tapered surfaces 58e are held in face-to-face contact with the inner surfaces defining the tapered portions 52j, respectively. The weight pieces 58a/ 58b are spaced from one another in the through-hole 52f.

Through-holes 58f are formed in the weight pieces 58a/ 58b. The through-hole 58f in the weight piece 58a is stepwise varied in the diameter, and the other through-hole 58f is partially tapered. The fastener 58c is implemented by a flat head bolt 58h and a hexagon nut 58j. The hexagon nut 58j is received in the stepwise varied through-hole 58f, and the flat head bolt 58h is inserted into the other through-holes 58f. The hexagon nut 58j is engaged with the threaded stem of the flat head bolt 58h. Thus, the weight pieces 58a/ 58b are fastened to the wood bar 52 by means of the fastener 58c.

Figure 13 shows the third modification 54C of the key balancer 54. The key balancer 54C includes a weight piece 60a, the weight piece 54a (not shown in figure 13) and a fastener 60c. The fastener 60c is implemented by the combination of flat head bolt 54e and hexagon nut 54f (not shown in figure 13). The weight piece 60a has a head 60d and a stem 60e. The flat head bolt 54e is embedded in the stem 60e through an insert molding, and the bolt 54e projects from the stem 60e as shown. A hexagonal recess 60f is formed in the head 60d, and a hexagonal wrench is to be snugly received in the hexagonal recess 60f. When the key balancer 54C is assembled with the wood bar 52, the weight piece 54a is inserted into the through-hole 52a/ 52b, and the hexagon nut 54f is received in the wide portion 54j. The weight piece 60a is partially inserted into the through-hole 52a/ 52b, and is rotated by a worker with the wrench. Then, the flat head bolt 54e is engaged with the hexagon nut 54f, and the weight pieces 60a/ 54a are fastened to the wood bar 52. Thus, the bolt 54e and hexagon nut 54f, i.e., the fastener serve as the anchor.

Figure 14 shows the fourth modification 54D of the key balancer 54. The key balancer 54D includes a weight piece 62a, the weight piece 54a and a fastener 62b. The fastener 62b is implemented by the combination of the bolt 54e and nut 54f. The hexagonal nut 54f is embedded in the weight piece 62a through the insert molding. The weight piece 62a has a head portion 62c and a stem portion 62d, and a hexagonal recess 62e is formed in the head portion 62c. The weight pieces 62a/ 54a are inserted into the through-hole 52a/ 52b, and the worker keeps the weight piece 62a stable with a wrench inserted into the hexagonal recess 62e. The worker drives the flat headed bolt 54e for rotation, and the bolt 54e is engaged with the nut 54f.

The weight pieces 60a/ 62a and the fastener 54e/ 54f may form the fifth modification of the key balancer 54. In this instance, a worker inserts the weight pieces 60a/ 62a into the through-hole 52a/ 52b, and drives one of the weight pieces 60a/ 62a for rotation with a wrench. The fifth modification is desirable, because the bolt and nut are not carelessly lost.

Figure 15 shows the sixth modification 54E of the key balancer 54. The key balancer 54E includes weight pieces 64a/ 64b and a fastener 64c. The fastener 64c are integral with the weight pieces 64a/ 64b as will be described hereinafter in detail. The weight piece 64a has a head portion 64c' and a stem portion 64d, and the other weight piece 64b is implemented by a disc 64e. The head portion 64c' and disc 64e are received in the wide portions 52d of the through-hole 52a/ 52b, respectively, and the stem portion 64d is inserted into the narrow portion 52f of the through-hole 52a/ 52b.

The fastener 64c is implemented by a projection 64f and a receiver 64h. The projection 64f has a stem 64j and a ring 64k. The stem 64j is formed with the ring 64k. The ring 64k extends along the periphery of the stem 64j. The receiver 64h is implemented by a cylinder 64m, and the cylinder 64m is open at one end to the outside. The outer diameter of the cylinder 64m is equal to the inner diameter of the narrow portion 52f of the through-hole 52a/52b, and the inner diameter of the cylinder 64m is equal to the outer diameter of the stem 64j. Plural slits 64n are formed in the cylinder 64m so that the cylinder 64m is radially outwardly widened. A ring-shaped groove 64p is formed along the inner surface defining the inner space of the cylinder 64m, and the ring 64k is to be received in the ring-shaped groove 64p.

When a worker is embedded in the wood bar 52, the worker inserts the weight pieces 64a/ 64b from both side surfaces of the wood bar 52 into the through-hole 52a/ 52b. The stem 64j advances into the inner space of the cylinder 64m, and the ring 64k is brought into contact with the cylinder 64m. The worker strongly pushes the weight pieces 64a/ 64b. Then, the ring 64k causes the cylinder 64m to be outwardly radially deformed so that the ring 64k reaches the ring-shaped groove 64p. The ring 64k is received in the ring-shaped groove 64p, and the cylinder 64m is recovered to the initial configuration. The ring 64k is hardly moved out of the ring-shaped groove 64p. Thus, the fastener 64c keeps the key balancer 54E in the through-hole 52a/52b stable.

When the worker disassembles the key balancer 54E, the worker inserts a pin 66a into a hole 66b formed in the weight piece 64b, and hits the head portion of the pin 66a. Then, the ring 64k is moved out of the ring-shaped groove 64p, and the projection 64f is separated from the receiver 64h. The worker takes out the weight pieces 64a/ 64b from the through-hole 52a/ 52b.

Figure 16 shows the seventh modification 54F of the key balancer 54. The key 52 is formed with a through-hole 52m instead of the through-hole 52a/52b. The through-hole 52m is increased in cross section from the middle point toward both ends thereof. Thus, the through-hole 52m is symmetrical with respect to a virtual cross section perpendicular to the middle point of the centerline thereof. The key balancer 54F includes weight pieces 68a/ 68b and a fastener 68c. The weight pieces 68a/ 68b is shaped in a frusto-conical configuration, and through-holes 68d/ 68e are formed in the weight pieces 68a/68b, respectively. The through-hole 68d is stepwise varied in diameter, and the other through-hole 68e is partially tapered. The flat headed bolt 54e and hexagon nut 54f serve as the fastener 68c, and are engaged with one another in the through-holes 68d/ 68e. When the weight pieces 68a/ 68b are inserted into the through-hole 52m, the weight pieces 68a/ 68b are held in face-to-face contact with the inner surface defining the through-hole 52m, and the bottom surfaces 68f are coplanar with the side surfaces of the wood bar 52. The weight pieces 68a/ 68b are spaced from each other in the through-hole 52m. The weight pieces 68a/ 68b are fastened to the wood bar 52 by means of the bolt and nut 54e/ 54f.

Figure 16 shows the eighth modification 54G of the key balancer 54. The wood bar 52 is formed with a through-hole 52n instead of the through-hole 52a/ 52b. Although the through-hole 52n is also tapered toward the side surfaces of the wood bar 52, the cross section is minimized at a certain point leftward offset from the middle point. For this reason, the tapered inner surface is asymmetrical with respect to a virtual cross section passing through the certain point on the centerline of the through-hole 52n, and the tapered inner surface on the left side of the virtual cross section is different in angle from the tapered inner surface on the right side.

The key balancer 54G includes weight pieces 70a/ 70b and a fastener 70c. The weight pieces 70a and 70b have respective frusto-conical configurations different from each other. The weight piece 70a is to be inserted into the left portion of the through-hole 52n, and the other weight piece 70b is to be received in the right portion of the through-hole 52n. When the weight pieces 70a/ 70b are inserted into the through-hole 52n, the weight pieces 70a/ 70b are held in face-to-face contact with the tapered inner surfaces defining the through-hole 52n, and the bottom surfaces 70d are coplanar with the side surfaces of the wood bar 52. The fastener 70c is implemented by the bolt 54e and nut 54f. When the bolt 54e is engaged with the nut 54f, the weight pieces 70a/ 70b are fastened to the wood bar 52.

Those modifications 54A/ 54B/ 54C/ 54D/ 54E/ 54F/ 54G achieve all the advantage of the key balancer 54.

### Third Embodiment

Turning to figure 18 of the drawings, a key 91 forming a part of a keyboard, which is incorporated in an acoustic piano. The acoustic piano is similar to that shown in figure 1 except the keys 91. For this reason, description is focused on the key 91, and the other component parts are specified by using references designating corresponding parts in figure 1.

The key 91 serves as a white key, and comprises a wood bar 92, a decorative plate 93 and key balancers 94. The wood bar 92 is made of Japanese spruce, which belongs to silver fir. Although a front portion and a rear portion are shown in figure 18, a vertical hole is formed in a middle portion of the wood bar 92, and a balance pin (not shown) passes through the vertical hole. The balance pin gives the fulcrum 3d to the white key 91 placed on the balance rail 3b.

The upper surface and front end surface of the front portion of the wood bar 92 are covered with the decorative plate 93. The decorative plate 93 is like an angle, and is adhered to the wood bar 92. The decorative plate 93 is made of synthetic resin, and the synthetic resin is colored in white.

Two through-holes 92a and 92b are formed in the front portion of the wood bar 92. The through-holes 92a/ 92b are in parallel, and each of the through-holes 92a/ 92b is open at both ends thereof on the side surfaces of the wood bar 92 to the outside. Each of the through-holes 92a/ 92b has a narrow portion 92c and wide portions 92d. As will be seen in figure 19, the wide portions 92d are exposed to the side surfaces of the wood bar 92, and are formed on both sides of the narrow portion 92c. In this instance, the wide portions 92 are equal in depth and diameter to one another. The key balancers 94 are embedded in the through-holes 92a/ 92b so as to exert the counter moment on the white key 91.

Each of the key balancers 94 includes a weight piece 96a and a fastener 96b. The weight piece 96a has a weight column piece 96c, a tube 96d and a pin 96e. The tube 96d is equal in diameter to the narrow portion 92c, and is flared at both end portions 96f. The flared end portions 96f are larger in diameter than the narrow portion 92c, and serves as the fastener 96b. On the other hand, the outer diameter of the weight column 96c is equal to or slightly less than the inner diameter of the tube 96d, and the length of the weight column 96c is equal to the length of the narrow portion 92c. Holes are formed in both tube and weight column 96c/ 96d, and are equal in diameter to or slightly less than the pin 96e.

The tube, weight column and pin 96d/ 96c/ 96e are assembled into the weight piece 96a as follows. The weight column and tube 96c/ 96d are drilled. The weight column 96c is inserted into the tube 96d, and the hole formed in the tube 96d is aligned with the hole formed in the weight column 96c. The pin 96e is driven into the holes so that the weight column 96c is fixed to the tube 96d by means of the pin 96e. Otherwise, the weight column 96c is inserted into the tube 96d, and the weight column and tube 96d/ 96d are concurrently drilled so that the holes are formed therein. The pin 96e is driven into the holes, and the weight column 96c is fixed to the tube 96d.

The weight column 96c is made of heavy metal except harmful metal such as lead and mercury. The heavy metal available for the weight column 96c is, by way of example, iron, brass, tungsten and sintered metal. Composite material is also available for the weight column 96c. The composite material contains the heavily metal and synthetic resin. Although any sort of non-harmful metal is available for the key weight column 96c, tungsten is preferable. Tungsten has the specific gravity of 19.3, and is heavier than lead. Even though the synthetic resin is mixed with tungsten, the composite material has the specific gravity as large as or larger than lead. The synthetic resin available for the composite material is, by way of example, thermosetting resin in the urethane series, polyester series, epoxy series, phenol series, urea series and melamine series or thermoplastic resin in the ABS (Acrylonitrile-Butadiene- Styrene) series and acrylic resin series. It is preferable to increase the amount of heavy metal of the composite material, because the weight column 96c exerts large counter moment on the key 91. In case where the weight column 96c is made of iron, the exposed surfaces of the weight column 96c are to be preserved.

The tube 96d is made of metal such as, for example, copper or brass in order to make the tube 96d sufficiently deformed. Otherwise, the tube 96d is made of composite material containing metal and synthetic resin. Any metal except the harmful metal such as lead and mercury is available for the composite material.

The fastener 96b is formed after the insertion of the tube/ weight column 96d/ 96c into the through-hole 92a as follows. Figure 20 shows the tube/weight column 96d/ 96c just inserted into the through-hole 92a. The tube 96d is straight, and the length of the tube 96d is approximately equal to the width of the wood bar 92. A pair of caulking bits 98a is prepared. The caulking bits 98a have respective tapered portions 98b, and the tapered portions 98b have the minimum diameter equal to the inner diameter of the tube 96d. A worker aligns the tapered portions 98b with the inner space of the tube 96d, and presses the caulking bits 98a against both ends of the tube 96d. The end portions are flared so that the weight piece 96a is caulked to the wood bar 92. Thus, the flared end portions 96f, i.e., the fastener serves as an anchor.

As will be understood from the foregoing description, the flared end portions 96f keep the weigh piece 96a stable in the wood bar 92. The weight pieces 96a are neither chattered in nor dropped out from the wood bar 92, and are easily assembled with the wood bar 92 through the caulking. The fastener is implemented by the flared end portions 96f of the tube 96d so that any part is not required for the fastening. As a result, the production cost is reduced.

The press fitting is not required for the key balancers 94. Any force is not exerted on the wood bar 92 in the direction of the thickness thereof, and, accordingly, the wood bar 92 is never cracked.

The first modification 94A of the key balancer 94 is shown in figure 21. The tube 96d is fixed to the weight column 96c by means of projections 99a formed by using a punch 102. The pin 96e is not required for the weight piece of the key balancer 94A, and the production cost is further reduced. Both end portions of the tube 96d are flared after insertion into the through-hole 92a. The tube 96d and the weight column 96c may be monolithic.

Figure 22 shows the second modification 94B of the key balancer 94. Straight through-holes 92r are formed in the wood bar 92, and the key balancer 94B is implemented by a weight column 103. A slit 104 is formed in the weight column 103, and semi-column portions 103a/ 103b are opposed to each other through the slit 104. A pair of semi-conical recess 105 is formed in the semi-column portions 103a/ 103b, and is narrower than a punch 106. The weight columns 103 are firstly inserted into the through-holes 92r, respectively. A worker aligns the punch 106 with the pair of semi-conical recess 105, and inserts the tip of the punch 106 into the pair of semi-conical recess 105. The worker hits the punch 106 so that the tip deeply inserted into the pair of semi-conical recess 105. The semi-column portions 103a/ 103b are plastically deformed, and are spaced from each other. The outer surfaces of the semi-column portions 103a/ 103b are pressed to the inner surface defining the through-hole 92r, and the weight column 103 is fastened to the wood bar 92. In this instance, the semi-column portions 103a/ 103b serves as an anchor.

It is preferable to direct the slit 104 in the vertical direction as shown in figure 22, because the wooden bars 92 are not cracked. In other words, the expanded semi-column portions 103a/ 103b do not exert the force in the direction of the thickness of the wooden bars 92, and the wooden bars 92 withstand the expanded semi-column portions 103a/ 103b.

In the first to third embodiments and their modifications, the action units 7, hammers 11 and the strings form parts of a tone generating system.

In the first to third embodiments and their modifications, the key balancers are embedded in the front portions of the wooden bars. However, the key balancers may be embedded in the rear portions of the wooden bars in order to vary the moment of inertia. In may models of upright pianos, the key-touch is dominated by the moment of inertia, and the key balancers are embedded in the rear portions of the wooded bars.

From the viewpoint of the key-touch, the term "moment" means both of the moment of force and the moment of inertia, and term "regulative moment" means the counter-moment and the factor for varying the moment of inertia.

Although particular embodiments of the present invention have been shown and described, it will be apparent to those skilled in the art that various changes and modifications may be made without departing from the spirit and scope of the present invention.

In case where the manufacturer does not make the side surfaces of the black/ white keys flat, straight through-holes are formed in the wooded bars. In other words, the wide portions such as, for example, 92d are not required, and the machining cost is reduced.

The keys 21, 51 and 91 are available for en electronic keyboard and a composite keyboard musical instrument. In the electronic keyboard, the wood bars 22, 52, 92 may be replaced with bars made of synthetic resin, and are swingably connected to a frame by means of pins. In the electronic keyboard, a key scanner, an information processing system, a tone generator and a sound system form in combination the tone generating system. The keys are periodically scanned by the key scanner to see whether or not a player depresses any one of the keys. The key scanner is connected to the key sensors for monitoring the keys, and supplies a key scanning signal representative of a key or keys depressed or released by a player. The key scanner is connected to the information processing system, and the information processing system analyzes the key scanning signal for specifying the key or keys. The information processing system produces music data codes representative of the tone or tones to be generated or decayed, and supplies the music data codes to the tone generator. The tone generator produces a digital tone signal on the basis of the music data codes, and converts the digital tone signal to an analog tone signal. The analog tone signal is supplied to the sound system, and electronic tones are produced from the analog tone signal.

## Claims

1. A keyboard musical instrument for generating tones, comprising:
a keyboard (1) including plural keys (21; 51; 91) used for specifying pitches of tones to be produced, applied with moments urging said plural keys (21; 51; 91) to rest positions thereof and having respective bars (22/ 52/ 92), each of said plural keys (21; 51; 91) having at least one key balancer (24; 24A; 24B; 24C; 24D; 54; 54A; 54B; 54C; 54D; 54E; 54F; 54G; 94; 94A; 94B) for applying a regulative moment to said each of said plural keys (21; 51; 91) for varying the moment, and
a tone generating system (7/ 11/ 12) connected to said plural keys (21; 51; 91), and generating said tones with said pitches;
said at least one key balancer (24; 24A; 24B; 24C; 24D; 54; 54A; 54B; 54C; 54D; 54E; 54F; 54G; 94; 94A; 94B) having a weight piece (24a; 26a; 30c; 31/ 32; 54a; 56a/ 56b; 58a/ 58b; 60a/ 58b; 58a/ 62a; 64a/ 64b; 68a/ 68b; 70a/70b; 96a; 96c/ 96d; 103) made of non-lead material and embedded in the bar (22; 52; 92) of said each of said plural keys and an anchor (24b; 26b; 28a/28b/ 28c; 30d; 31b/ 32a; 54b; 56a/ 56e; 58c; 54e/ 54f; 64c; 96f; 103a/ 103b) for fixing said weight piece to said bar of said each of said plural keys;
**characterizing in that**
at least one through-hole (52a/ 52b) is formed in said bar (52), and said weight piece (54a) is fastened to said bar (52) by means of said anchor (54b).

2. The keyboard musical instrument as set forth in claim 1, in which said at least one through-hole (52a/ 52b) has a narrow portion (52c) and wide portions (52d) continuous to both ends of said narrow portion (52c) and exposed to side surfaces of said bar (52), and said weight piece (54a) has a pair of weight sub-pieces each received in one of said wide portions (52d) and a part of said narrow portion (52c) so that said anchor (54b) fastens said weight sub-pieces (54a) to said bar (52).

3. The keyboard musical instrument as set forth in claim 2, in which said anchor is constituted by a bolt (54e) and a nut (54f), and said bolt (54e) is engaged with said nut (54f) in through-holes (54h/ 54j/ 54k/ 54m) formed in said weight sub-pieces (54a), respectively.

4. The keyboard musical instrument as set forth in claim 2, in which said anchor is constituted by a bolt (56c) and a female screw (56e) formed in one of said weight sub-pieces (56b).

5. The keyboard musical instrument as set forth in claim 3, in which said bolt (54e) is integral with one of said weight sub-pieces (60a).

6. The keyboard musical instrument as set forth in claim 3, in which said nut (54f) is integral with one of said weight sub-pieces (62a),

7. The keyboard musical instrument as set forth in claim 2, in which said anchor includes a projection (64j) formed with a ring (64k) extending around an outer surface thereof and a cylinder (64m) formed with a ring-shaped groove (64p) and slits (64n), and said projection (64j) is received in an inner space of said cylinder (64m) so that said ring (64k) is received in said ring-shaped groove (64p).

8. The keyboard musical instrument as set forth in claim 1, in which said at least one through-hole (52m; 52n) is increased in cross section from a certain point on the centerline thereof toward both ends, and said weight piece includes a pair of weight sub-pieces (68a/ 68b; 70a/ 70b) having respective cross sections increased in cross section so that said weight sub-pieces (68a/68b; 70a/ 70b) are received in said at least one through-hole (52m; 52n)

9. A keyboard musical instrument for generating tones, as set forth in claim 1, in which said weight piece (96a) is caulked to said bar by means of said anchor (96b).

10. The keyboard musical instrument as set forth in claim 9, in which said at least one through-hole (92a/ 92b) has a narrow portion (92c) and wide portions (92d) continuous to both ends of said narrow portion (92c) and exposed to side surfaces of said bar (92), said weight piece has a tube member (96d) received in said at least one through-hole (92a/ 92b) and a core member (96c) received in said tube member (96d), and both end portions (96f) of said tube member (96d) is flared so as to be caulked to said bar (92).

11. The keyboard musical instrument as set forth in claim 10, in which said core member (96c) is fixed to said tube member (96d) by means of a pin (96e).

12. The keyboard musical instrument as set forth in claim 10, in which said tube member (96d) is partially deformed so that said tube member (96d) is caulked to said core member (96c).

13. The keyboard musical instrument as set forth in claim 9, in which said weight piece (103) has plural parts (103a/ 103b) spaced from one another by means of at least one slit (104), and said plural parts (103a/ 103b) are expanded in said at least one through-hole (92a/ 92b) so as to serve as said anchor.

14. A process for fabricating a key incorporated in a keyboard musical instrument, comprising the steps of:
a) preparing a bar (92) and a weight piece (96a); and
b) securing said weight piece (96a) to said bar (92),
**characterized in that**
said step a) includes the sub-step of forming a substantially straight through-hole in said bar (92) and said weight piece (92a) into a shape having at least a substantially straight tube portion (96d) roughly equal in diameter to said substantially straight through-hole and a core portion (96c) disposed in said substantially straight tube portion (96d),
and **in that**
said step b) includes the sub-steps of
b-1) inserting said weight piece (92a) into said substantially straight through-hole and
b-2) caulking both end portions (96f) of said substantially straight tube portion (96d) to said side portions of said bar (92) so that both of said both end portions (96f) and said side portions are flared.

## Patentansprüche

1. Ein Tastenmusikinstrument zum Erzeugen von Tönen, welches folgendes aufweist:
Eine Tastatur (1) einschließlich mehreren Tasten (21; 51; 91), welche zum Spezifizieren von Tonhöhen von Tönen, welche erzeugt werden sollen, verwendet wird, angewandt mit Momenten, welche die Vielzahl von Tasten (21; 51; 91) auf hohe Positionen davon drängen und jeweilige Stäbe (22/ 52/ 92) haben, wobei jede der Vielzahl von Tasten (21; 51; 91) mindestens einen Tastenausgleicher (24; 24A; 24B, 24C, 24D, 54; 54A, 54C, 54D; 54E; 54F; 54G; 94; 94A; 94B) zum Anwenden eines regulierenden Moments auf jede der Vielzahl von Tasten (21; 51; 91) zum Variieren des Moments hat, und ein Tonerzeugungssystem (7/ 11/12), welches mit der Vielzahl von Tasten (21; 51; 91) verbunden ist, und die Töne mit den Tonhöhen erzeugt;
wobei der mindestens eine Tastenausgleicher (24; 24A; 24B, 24C, 24D, 54; 54A, 54C, 54D; 54E; 54F; 54G; 94; 94A; 94B) ein Gewichtstück (24a; 26a; 30c; 31/ 32; 54a; 56a/ 56b; 58a/ 58b; 60a/ 58b; 58a/62a; 64a/ 64b; 68a/ 68b; 70a/ 70b; 96a; 96c/ 96d; 103) hat, welches aus nicht bleihaltigem Material hergestellt ist und in dem Stab (22; 52; 92) von jeder der Vielzahl von Tasten eingebettet ist, und einen Anker oder eine Verankerung (24b; 26b; 28a/ 28b/ 28c; 30d; 31 b/ 32a; 54b; 56a/ 56e; 58c; 54e/ 54f; 64c; 96f; 103a/ 103b) zum Fixieren des Gewichtstücks an dem Stab von jeder der Vielzahl von Tasten;
**dadurch gekennzeichnet, dass**
mindestens ein Durchgangsloch (52a/ 52b) in dem Stab (52) ausgebildet ist, und das Gewichtsstück (54a) an dem Stab (52) durch den Anker (54b) befestigt ist.

2. Das Tastenmusikinstrument gemäß Anspruch 1, wobei das mindestens eine Durchgangsloch (52a/ 52b) einen schmalen Teil (52c) und weite Teile (52d) kontinuierlich an beiden Enden des schmalen Teils (52c) und zu Seitenoberflächen des Stabs (52) zugewandt hat, und wobei das Gewichtstück (54a) ein Paar von Gewichts-Teilstücken hat, welche jeweils in einem der weiten Teile (52d) und einem Teil des schmalen Teils (52c) derart aufgenommen sind, dass der Anker (54b) die Gewichts-Teilstücke (54a) an dem Stab (52) befestigt.

3. Das Tastenmusikinstrument gemäß Anspruch 2, wobei der Anker durch einen Bolzen (54e) und eine Mutter (54f) ausgebildet ist, und wobei der Bolzen (54e) mit der Mutter (54f) in Durchgangslöchern (54h/ 54j/ 54k/ 54m) in Eingriff kommt, welche in den Gewichts-Teilstücken (54a) jeweils ausgebildet sind.

4. Das Tastenmusikinstrument gemäß Anspruch 2, wobei der Anker durch einen Bolzen (56c) und ein Innengewinde (56e), welche in einer der Gewichts-Teilstücke (56b) ausgebildet ist, ausgebildet ist.

5. Das Tastenmusikinstrument gemäß Anspruch 3, wobei der Bolzen (54e) integral mit einem der Gewichts-Teilstücke (60a) ist.

6. Das Tastenmusikinstrument gemäß Anspruch 3, wobei die Mutter (54f) integral mit einem der Gewichts-Teilstücke (62a) ist.

7. Das Tastenmusikinstrument gemäß Anspruch 2, wobei der Anker einen Vorsprung (64j) beinhaltet, welcher mit einem Ring (64k) ausgebildet ist, welcher sich um die äußere Oberfläche davon erstreckt, und einen Zylinder (64m), welcher mit einer ringförmigen Nut (64p) und Schlitzen (64n) ausgebildet ist, und wobei der Vorsprung (64j) in einem Innenraum von dem Zylinder (64m) derart aufgenommen wird, dass der Ring (64k) in der ringförmigen Nut (64p) aufgenommen wird.

8. Das Tastenmusikinstrument gemäß Anspruch 1, wobei das mindestens eine Durchgangsloch (52m; 52n) im Querschnitt von einem bestimmten Punkt auf der Mittenlinie davon in Richtung von beiden Enden vergrößert wird, und wobei das Gewichtstück ein Paar von Gewichts-Teilstücken (68a/ 68b; 70a/ 70b) beinhaltet, welche jeweilige Querschnitte mit vergrößertem Querschnitt derart haben, dass die Gewichts-Teilstücke (68a/ 68b; 70a/ 70b) in dem mindestens einen Durchgangsloch (52m; 52n) aufgenommen sind.

9. Ein Tastenmusikinstrument zum Erzeugen von Tönen gemäß Anspruch 1, in welchem das Gewichtstück (96a) mit dem Stab durch den Anker (96b) verstemmt ist.

10. Das Tastenmusikinstrument gemäß Anspruch 9, wobei das mindestens eine Durchgangsloch (92a/ 92b) einen schmalen Teil (92c) und weite Teile (92d) hat, welche an beiden Enden des schmalen Teils (92c) kontinuierlich sind und zu Seitenoberflächen des Stabs (92) ausgerichtet sind, wobei das Gewichtstück ein Röhrenglied (96d) hat, welches in dem mindestens einen Durchgangsloch (92a/ 92b) aufgenommen wird, und ein Kernglied (96c), welches in dem Röhrenglied (96d) aufgenommen wird, und wobei beide Endteile (96f) des Röhrenglieds (96d) derart aufgeweitet sind, dass sie mit dem Stab (92) verstemmt sind.

11. Das Tastenmusikinstrument gemäß Anspruch 10, wobei das Kernglied (96c) an dem Röhrenglied (96d) durch einen Stift (96e) befestigt ist.

12. Das Tastenmusikinstrument gemäß Anspruch 10, wobei das Röhrenglied (96d) teilweise derart deformiert ist, dass das Röhrenglied (96d) an dem Kernglied (96c) verstemmt ist.

13. Das Tastenmusikinstrument gemäß Anspruch 9, wobei das Gewichtsstück (103) mehrere Teile (103a/ 103b) hat, welche voneinander durch mindestens einen Schlitz (104) beabstandet sind, und wobei die mehreren Teile (103a/ 103b) in dem mindestens einen Durchgangsloch (92a/ 92b) derart erweitert sind, dass sie als der Anker dienen.

14. Ein Prozess zum Herstellen eines Tastenmusikinstruments, welcher folgende Schritte aufweist:
a) Vorbereiten eines Stabs (92) und eines Gewichtstücks (96a); und
b) Sichern des Gewichtstücks (96a) an dem Stab (92),
**dadurch gekennzeichnet, dass**
der Schritt a) den Teilschritt des Ausbildens eines im Wesentlichen geraden Durchgangslochs in dem Stab (92) und des Gewichtstücks (92a) in eine Form beinhaltet, welche mindestens einen im Wesentlichen geraden Röhrenteil (96d) ungefähr gleich im Durchmesser mit dem im Wesentlichen geraden Durchgangsloch, und einen Kernteil (96c) hat, welcher in dem im Wesentlichen geraden Röhrenteil (96d) angeordnet ist,
und **dadurch**, dass
der Schritt b) folgende Teilschritte aufweist:
b-1) Einfügen des Gewichtstücks (92a) in das im Wesentlichen gerade Durchgangsloch und
b-2) Verstemmen von beiden Endteilen 96f des im Wesentlichen geraden Röhrenteils (96d) zu den Seitenteilen des Stabs 92 derart, dass beide der beiden Endteile (96f) und der Seitenteile erweitert werden.

## Revendications

1. Instrument de musique à clavier pour produire des sons, comprenant :
un clavier (1) comprenant une pluralité de touches (21, 51, 91) utilisées pour spécifier des hauteurs de son à produire, soumises à des moments sollicitant la pluralité de touches (21, 51, 91) vers des positions de repos et comportant des barres respectives (22, 52, 92), chacune de la pluralité de touches (21, 51, 91) comprenant au moins un équilibreur de touches (24, 24A, 24B, 24C, 24D, 54, 54A, 54B, 54C, 54D, 54E, 54F, 54G, 94, 94A, 94B) pour appliquer un moment régulateur à chacune de la pluralité de touches (21, 51, 91) pour modifier le moment ; et
un système de génération de sons (7, 11, 12) connecté à la pluralité de touches (21, 51, 91) et produisant des sons ayant lesdites hauteurs ;
ledit au moins un équilibreur de touches (24, 24A, 24B, 24C, 24D, 54, 54A, 54B, 54C, 54D, 54E, 54F, 54G, 94, 94A, 94B) comprenant un contrepoids (24a ; 26a ; 30c ; 31, 32 ; 54a ; 56a, 56b ; 58a, 58b ; 60a, 58b ; 58a, 62a ; 64a, 64b ; 68a, 68b ; 70a, 70b ; 96a ; 96c, 96d ; 103) en un matériau non conducteur et logé dans la barre (22, 52, 92) de chacune de la pluralité de touches et une ancre (24b ; 26b ; 28a, 28b, 28c ; 30d ; 31b, 32a ; 54b ; 56a, 56e ; 58c ; 54e, 54f ; 64c ; 96f ; 103a, 103b) pour fixer le contrepoids à la barre de chacune de la pluralité de touches ;
**caractérisé en ce que** au moins un trou traversant (52a, 52b) est formé dans ladite barre (52) et le contrepoids (54a) est fixé à la barre (52) au moyen de ladite ancre (54b).

2. Instrument de musique à clavier selon la revendication 1, dans lequel ledit au moins un trou traversant (52a, 52b) comporte une partie étroite (52c) et des parties larges (52d) continues aux deux extrémités de la partie étroite (52c) et exposées aux surfaces latérales de la barre (52), et le contrepoids (54a) comporte une paire de sous-contrepoids dont chacun est reçu dans l'une des parties larges (52d) et une partie de la partie étroite (52c) de sorte que l'ancre (54b) lie les sous-contrepoids (54a) à la barre (52).

3. Instrument de musique à clavier selon la revendication 2, dans lequel l'ancre est constituée d'un boulon (54e) et d'un écrou (54f) et le boulon (54e) entre dans l'écrou (54f) dans les trous traversants (54h, 54j, 54k, 54m) formés dans les sous-contrepoids (54a), respectivement.

4. Instrument de musique à clavier selon la revendication 2, dans lequel l'ancre est constituée d'un boulon (56c) et d'une vis femelle (56e) formée dans l'un des sous-contrepoids (56b).

5. Instrument de musique à clavier selon la revendication 3, dans lequel le boulon (54e) est d'une seule pièce avec l'un des sous-contrepoids (60a).

6. Instrument de musique à clavier selon la revendication 3, dans lequel l'écrou (54f) est d'une seule pièce avec l'une des sous-contrepoids (62a).

7. Instrument de musique à clavier selon la revendication 2, dans lequel l'ancre inclut une partie en saillie (64j) comportant une bague (64k) s'étendant autour de sa surface externe et un cylindre (64m) comportant une rainure en anneau (64p) et des fentes (64n), et la partie en saillie (64j) est reçue dans un espace interne du cylindre (64m) de sorte que la bague (64k) est reçue dans la rainure en forme d'anneau (64p).

8. Instrument de musique à clavier selon la revendication 1, dans lequel ledit au moins un trou traversant (52m, 52n) a une section accrue à partir d'un certain point de son axe vers ses deux extrémités et le contrepoids comprend deux sous-contrepoids (68a, 68b ; 70a, 70b) ayant des sections respectives qui croissent de sorte que les sous-contrepoids (68a, 68b ; 70a, 70b) sont reçus dans ledit au moins un trou traversant (52m, 52n).

9. Instrument de musique à clavier selon la revendication 1, dans lequel le contrepoids (96a) est maté à la barre par ladite ancre (96b).

10. Instrument de musique à clavier selon la revendication 9, dans lequel ledit au moins un trou traversant (92a, 92b) comporte une partie étroite (92c) et des parties larges (92d) continues aux deux extrémités de la partie étroite (92c) et exposées aux surfaces latérales de la barre (92), le contrepoids comprend un élément de tube (96d) reçu dans ledit au moins un trou traversant (92a, 92b) et un élément de coeur (96c) reçu dans l'élément de tube (96d), et les deux parties d'extrémité (96f) de l'élément de tube (96d) sont évasées pour être matées à la barre (92).

11. Instrument de musique à clavier selon la revendication 10, dans lequel l'élément de coeur (96c) est fixé à l'élément de tube (96d) par une tige (96e).

12. Instrument de musique à clavier selon la revendication 10, dans lequel l'élément de tube (96d) est partiellement déformé de sorte que l'élément de tube (96d) est maté sur l'élément de coeur (96c).

13. Instrument de musique à clavier selon la revendication 9, dans lequel le contrepoids (103) comprend plusieurs parties (103a, 103b) espacées les unes des autres par au moins une fente (104) et lesdites plusieurs parties (103a, 103b) s'étendent dans ledit au moins un trou traversant (92a, 92b) de façon à servir d'ancre.

14. Procédé de fabrication d'une touche incorporée à un instrument de musique à clavier, comprenant les étapes suivantes :
a) préparer une barre (92) et un contrepoids (96a) ; et
b) fixer le contrepoids (96a) à la barre (92) ;
**caractérisé en ce que** l'étape a) comprend la sous-étape consistant à former un trou traversant sensiblement rectiligne dans la barre (92) et le contrepoids (92a) selon une forme comportant au moins une partie de tube sensiblement droit (96d) de diamètre en gros égal au trou traversant sensiblement droit et une partie de coeur (96c) disposée dans la partie de tube sensiblement droit (96d) ;
et **en ce que** l'étape b) comprend les sous-étapes suivantes :
b-1) insérer le contrepoids (92a) dans le trou traversant sensiblement droit ; et
b-2) mater les deux parties d'extrémité (96f) de la partie de tube sensiblement droit (96d) aux parties latérales de la barre (92) de sorte que les deux parties d'extrémité (96f) et des parties latérales sont évasées.
